# FASCICULE DE BREVET EUROPEEN

(11) **EP 2 791 313 B1**
(45) Date de publication et mention de la délivrance du brevet: **06.09.2017**
(21) Numéro de dépôt: 12818796.0
(22) Date de dépôt: 10.12.2012
(51) Int. Cl.: C12N 1/04, A21D 8/04, A61K 36/064, A61K 35/74, A61K 35/744, A61K 35/741, A61K 35/745, A61K 35/747, A23L 33/135, A23L 33/14

(54) **COMPOSITION COMPRENANT UNE BIOMASSE MICROBIENNE ACTIVE**
ZUSAMMENSETZUNG MIT EINER AKTIVIERTEN MIKROBIELLEN BIOMASSE
COMPOSITION COMPRISING AN ACTIVATED MICROBIAL BIOMASS

(30) Priorité: 16.12.2011 FR 1103902
(43) Date de publication de la demande: 22.10.2014
(73) Titulaire: Lesaffre et Compagnie, 75001 Paris (FR)
(72) Inventeur: LEJEUNE, Pascal, 59200 Tourcoing (FR); SOUICI, Jean-Bernard, 59940 Estaires (FR)
(74) Mandataire: Cabinet Plasseraud
(86) Numéro de dépôt international: PCT/FR2012/052859
(87) Numéro de publication internationale: WO 2013/088045

(56) Documents cités:
- EP-A1- 0 159 891
- EP-A1- 0 636 692
- EP-A1- 1 514 553
- WO-A1-01/68808
- WO-A1-2011/004375

## Description

L'invention se rapporte au domaine des compositions alimentaires comportant une biomasse microbienne, à un procédé pour leur préparation ainsi qu'à des activateurs de fermentation ou « starters », et plus particulièrement au domaine de la panification, de la laiterie et/ou des compléments alimentaires de type « probiotiques » comportant de telles compositions.

La présente invention concerne d'une manière générale une composition améliorée produite par un procédé de pulvérisation - encore appelé ci-après « sprayage » - d'un fort taux de biomasse microbienne, en particulier de biomasse bactérienne, sur un support.
Ledit support de la composition selon l'invention est notamment un support de type granulaire (granules ou sphérules par exemple), spécialement apte à maintenir une forte viabilité de la biomasse microbienne et une bonne conservation de cette viabilité dans le temps (préservation/persistance de la viabilité). Ladite composition, qui comporte des microorganismes vivants, en particulier des bactéries pulvérisées sur ledit support, dans les conditions habituelles d'utilisation de l'art est généralement mise en oeuvre en tant que starter/levain sous forme de poudres sèches.

Le domaine technique visé dans la présente invention est en particulier celui d'une composition séchée selon un procédé perfectionné et adapté à la production d'une biomasse microbienne vivante sous forme sèche à partir d'un support de type granulaire apte à être enrobé de manière régulière par une biomasse microbienne concentrée, ladite biomasse pouvant représenter de 10 à 30 % en matière sèche de la matière sèche totale du support enrobé.

Les deux grandes techniques de conservation de biomasse bactérienne vivante sous forme sèche sont la lyophilisation et la pulvérisation sur support.

EP 0636692 A1 de la demanderesse décrit une « Biomasse stable à base de cellules de levures et de bactéries lactiques et procédé de préparation ».

La lyophilisation est une technique chère, complexe et les rendements de séchage sont parfois faibles en fonction des souches et des techniques utilisées.

La pulvérisation ou sprayage de bactéries sur support est également connu de EP 0 636 692 A1. Mais ces techniques restent limitées quant à la quantité de biomasse que l'on peut déposer sur le support [inférieur à 0,5 % MS P/P dans le cas de EP 0 636 692 A1, au-delà de cette valeur on constate l'agglomération (mottage) de la poudre et une perte de viabilité en conservation] ce qui limite en conséquence les performances des compositions antérieures ainsi produites.

Les biomasses microbiennes commercialisées aujourd'hui comme starters pour levains sont obtenues soit par sprayage, soit par mélange d'une grande quantité de lyophilisat et de granules de levure, soit par mélange d'une grande quantité de lyophilisat avec un support diluant et elles sont conservées en absence d'oxygène à -20°C, pour avoir une durée de vie de 2 ans. En l'absence de ces conditions de conservation, la durée d'utilisation des starters n'excède pas 3 mois.

Les opérations de mélange, la nécessité d'une atmosphère sans oxygène, l'utilisation d'un film d'emballage étanche type « quadriplex » et le mode de conservation sont autant de paramètres qui restent encore difficiles à maîtriser, dans certains pays en particulier en ce qui concerne le respect de la chaine du froid. Il s'ensuit des problèmes de qualité sans parler des questions de coûts industriels et environnementaux à prendre en compte.
Se pose également un autre problème qui est récurrent et est lié à la forme actuelle des starters pour levains, à savoir, celui de l'existence d'un important temps de latence lors de l'ensemencement de la farine du levain, provoquant à la fois un allongement des temps de fermentation, une variabilité de la durée du levain et un risque de développement de la flore non contrôlée de la farine.

L'ensemble de ces problèmes limite le développement de l'application des starters/levains en panification alors que leur utilisation permet la production de pains de haute qualité aromatique et nutritionnelle.

Plus récemment, la biomasse bactérienne vivante a pu aussi être utilisée en tant que probiotique, avec les problèmes liés d'une part à la nécessité d'une forte concentration en biomasse vivante et d'autre part à la survie des microorganismes après le passage de l'estomac et de son pH fortement acide.

Les inventeurs ont par ailleurs observé que:
- les compositions de l'état de la technique présentent une hétérogénéité en raison de la présence d'agglomérats avec pour conséquence une difficulté de manipulation de ces compositions formant agglomérats ;
- les compositions antérieures présentent des temps de latence importants entraînant des temps de levains importants - typiquement de 16 h à 24 h - et nécessitant donc la mise en oeuvre d'installations de grandes dimensions pour effectuer ces fermentations ; ces temps importants augmentent par ailleurs sensiblement le risque de développement d'une flore indésirable pouvant générer des goûts parasites, des problèmes de panification voire le développement de bactéries pathogènes ;
- les compositions antérieures telle que décrites dans le brevet EP0636692A1 permettent d'atteindre, immédiatement après leur production, donc sans conservation, un pH de 5,7 en 3 h en test d'acidification, ce qui est insuffisant ; une acidification pour atteindre un pH typiquement inférieur à 5,4 après un an de conservation à 20°C est recherchée.

### RESUME DE L'INVENTION

La présente invention entend résoudre les problèmes énoncés ci-dessus et surmonter les difficultés rappelées ici et rencontrées avec les compositions antérieures.
Les inventeurs ont montré qu'il existe en effet encore le besoin d'une composition de produits secs à base d'un support apte à être enrobé et autorisant qu'on y pulvérise une très forte concentration de bactéries vivantes, une telle composition pouvant être utilisée notamment en tant qu'activateur de fermentation / starter ou probiotique.
Une telle composition est améliorée en ce sens qu'elle doit permettre une protection effective des bactéries vivantes pulvérisées sur ledit support vis-à-vis en particulier d'une série de « stress » du type « stress température » - le starter étant conservé à une température positive voire à température ambiante, « stress acide » - en particulier dans l'application probiotique, il correspond à la résistance au pH gastrique - et « stress oxygène » - lors de la conservation à l'air.

Aussi la présente invention vient satisfaire ce besoin longtemps ressenti d'une composition présentant les qualités rappelées ci-dessus.

La présente invention décrit une composition comprenant un support apte à être enrobé de manière régulière par une biomasse microbienne, de préférence très fortement concentrée, ladite biomasse représentant des pourcentages très élevés de bactéries en matière sèche de la matière sèche totale du support enrobé.

L'invention décrit plus particulièrement une composition comprenant un support apte à être enrobé de manière régulière par une biomasse microbienne, ladite biomasse représentant de 10 à 30 % en matière sèche de la matière sèche totale du support enrobé.

L'invention décrit également un procédé de préparation d'une composition comprenant les étapes suivantes consistant à :
i- introduire un support apte à être enrobé dans un mélangeur traversé par un courant ascendant d'air chaud,
ii- pulvériser une suspension de biomasse microbienne comprenant plus de 5 % en matière sèche de bactéries (P/P),
iii- sécher par courant d'air chaud dont la température et le débit sont fixés de manière à ce que la température de ladite composition ne dépasse pas 40°C,
iv- récupérer un support enrobé, et
v- procurer ladite composition.

L'invention décrit encore un « **Activateur de fermentation** » ou « **Starter** » comportant la composition de l'invention ou telle qu'obtenue selon le procédé de l'invention, en particulier de type ferment panaire ou de type ferment de vinification ou encore de type ferment laitier.

L'invention décrit également un « **Probiotique** » comportant la composition de l'invention ou telle qu'obtenue selon le procédé de l'invention.

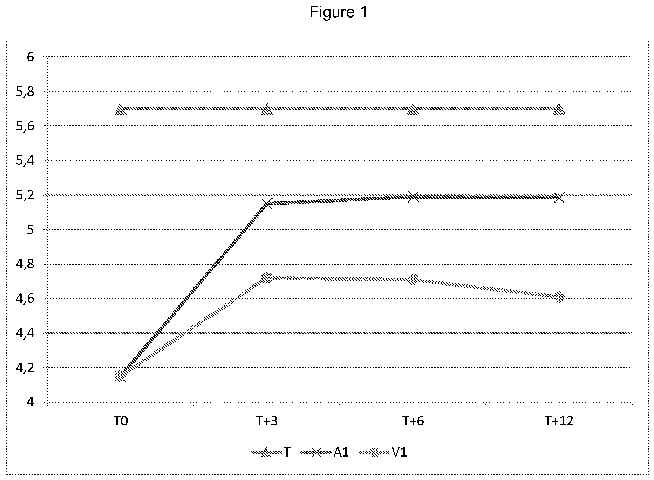

La Figure 1 représente les résultats d'un test d'acidification réalisé avec une composition préférée de l'invention, le SPRAY_A, qui a été conservée 1 an à 20°C sous air (A1) et sous vide (V1) par comparaison à ceux obtenus avec une composition témoin (T) du commerce et conservée dans les meilleures conditions, à savoir -20°C sous vide. La Figure 1 exprime l'évaluation du pH en fonction du temps de conservation en mois.

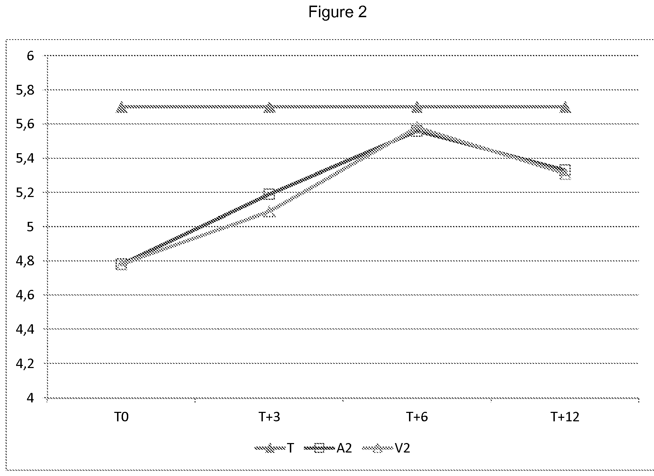

La Figure 2 représente les résultats d'un test d'acidification réalisé avec une composition préférée de l'invention, le SPRAY_C dit essai de « sursprayage » qui a été conservée 1 an à 20 °C sous air (A2) et sous vide (V2) par comparaison à ceux obtenus avec un témoin (T) du commerce et conservé dans les meilleures conditions, à savoir -20°C sous vide. La Figure 2 exprime l'évaluation du pH en fonction du temps de conservation en mois.

### DESCRIPTION DETAILLEE DE L'INVENTION

L'invention a pour objet une composition telle que décrite à l'une quelconque des revendications 1 à 13.
Il est décrit dans la présente invention une composition comprenant un support apte à être enrobé de manière régulière par une biomasse microbienne, ladite biomasse représentant de 10 à 30 % en matière sèche de la matière sèche totale du support enrobé.

Les inventeurs de la présente invention se sont particulièrement attachés à développer une composition améliorée qui renferme une biomasse qui soit stable et performante et ont mis au point un procédé de préparation spécifique de ladite composition de sorte qu'elle réponde favorablement à des critères particulièrement discriminants et significatifs dans les domaines d'application visés et cités ci-dessus.

Les performances des compositions selon l'invention sont contrôlées par des tests spécifiques que les inventeurs ont mis au point pour certains d'entre eux et utilisés au cours de leurs recherches.

Ces tests, ci-après dénommés « tests d'évaluation », sont centrés autour de la viabilité et des performances acidifiantes des compositions en particulier :
- le test d'acidification par observation de la baisse du pH en 3 h d'un milieu à base de maltose + sels minéraux réalisée à 35°C après ensemencement d'une quantité déterminée de la composition ;
- la détermination de la viabilité bactérienne par mesure du nombre de colonies bactériennes se développant sur un milieu standard par étalement d'une solution contenant une quantité connue de la composition.

Le temps d'exposition à la chaleur au cours de la fabrication de la composition de l'invention est aussi un paramètre qui est pris en compte.

Les inventeurs ont ensuite effectué des mesures des paramètres de conservation de ladite composition après sa production conformément au procédé de la présente invention définissant ainsi des pourcentages de préservation/persistance de la viabilité et des performances acidifiantes.

Une série de tests de conservation a ainsi été réalisée sur la composition de l'invention dans différentes conditions notamment de température, d'exposition à l'air et de durée. En particulier, des essais des performances de ladite composition sont effectués. Ce sont en particulier les tests suivants :
- sous air et sous vide à -20°C (température de stockage de référence pour ce type de produit)
- sous air et sous vide à 4°C, et
- sous air et sous vide à température ambiante (20°C).

Ont également été déterminées :
- la quantité finale de matière sèche ajoutée de type bactéries dans la composition par granule formé (MS bactéries/g de composition). (Rappel : cette quantité dépend du taux de sprayage) ;
- l'humidité finale de la composition de l'invention ;
- la distribution granulométrique (granulométrie laser) du support de la composition selon l'invention.

La composition décrite dans l'invention présente les caractéristiques complémentaires ou alternatives suivantes :
- de préférence, ladite biomasse représente de 13 à 26 % en matière sèche de la matière sèche totale du support enrobé ;
- le support enrobé comprend avantageusement en outre une couche de protection comprenant au moins un composé choisi parmi les hydrocolloïdes, les gommes, les dextrines notamment les maltodextrines, les poly, di ou monosaccharides et leurs dérivés ;
- ledit support se présente de manière préférée sous la forme de granules et/ou de sphérules ;
- lesdites sphérules présentent de préférence un diamètre moyen d(0,5) compris entre 150 et 2000 µm, de préférence entre 150 µm et 1200 µm et encore plus préférentiellement entre 150 µm et 700 µm ;
- lesdites granules présentent avantageusement une longueur moyenne comprise entre 1,8 et 2,2 mm et un diamètre moyen compris entre 0,4 et 0,7 mm ;
- ledit support est de manière avantageuse choisi parmi les levures sèches actives et les semoules de céréales ;
- lesdites levures présentent de préférence un taux de matière sèche supérieur à 94 %, de préférence supérieur à 96 %, lesdites granules de levures ayant avantageusement un effet bénéfique en tant qu'absorbeur d'humidité ;
- lesdites semoules présentent de préférence un taux d'humidité inférieur ou égal à 8 %.

De manière avantageuse, lesdites levures sont du genre *Saccharomyces* comprenant notamment l'espèce *S*. *chevalieri.*
La biomasse microbienne comprend au moins des bactéries choisies dans le groupe constitué par des bactéries appartenant à l'un des genres suivants *Lactobacillus, Pediococcus, Streptococcus, Leuconostoc, Lactococcus, Bifidobacterium, Propionibacterium,* et *Bacillus.*
Lesdites bactéries sont choisies avantageusement dans le groupe comprenant : *Lactobacillus plantarum, Lactobacillus brevis, Lactobacillus casei, Lactobacillus paracasei, Lactobacillus sanfrancisco, Lactobacillus amylovorum, Lactobacillus kefir, Lactobacillus pentosaceus, Lactobacillus acidilactici, Lactobacillus rhamnosus, Leuconostoc oenos, Leuconostoc mesenteroïdes et Bacillus subtilis.*

Le support enrobé comprend de préférence en outre une couche qui consiste en une crème de levure y pulvérisée, de préférence une crème de *S*. *chevalieri,* formant couche protectrice. Il est observé alors une amélioration dans la protection des bactéries lors du test de conservation sous air.
La composition présente avantageusement un taux de mortalité bactérien inférieur ou égal à 0,5 Log U.F.C./g après une conservation d'un an à une température de 20°C sous vide et/ou après une conservation d'un an à une température de 4°C sous air.

De préférence la composition présente, après 1 an de conservation à une température positive voire à température ambiante, lors du test d'acidification sur milieu au maltose, une diminution de pH de 6,5 à au moins 5,7 après seulement 3 h.
Avantageusement, la composition selon l'invention et comportant un support qui consiste en des levures sèches actives comporte en outre au moins un additif, ou auxiliaire technologique, dit de séchage lequel est de manière avantageuse choisi dans le groupe comprenant des mono et diglycérides d'acides gras modifiés, des esters d'acides gras et de sorbitan, tel que le monostéarate de sorbitan, des esters d'acides gras et de glycérol, des esters d'acides gras et de propylène glycol, de la méthylcellulose, de la carboxyméthylcellulose, de l'hydroxypropylcellulose et/ou un mélange de ces derniers.

La présente invention a encore pour objet un procédé de préparation tel que décrit à l'une quelconque des revendications 14 à 16.
Le procédé de préparation décrit dans l'invention comprend les étapes suivantes consistant à :
i- introduire un support apte à être enrobé dans un mélangeur traversé par un courant ascendant d'air chaud,
ii- pulvériser une suspension de biomasse microbienne comprenant plus de 5 % en matière sèche de bactéries (P/P),
iii- sécher par courant d'air chaud dont la température et le débit sont fixés de manière à ce que la température de ladite composition ne dépasse pas 40°C,
iv- récupérer un support enrobé, et
v- procurer ladite composition.

Le mélangeur qui est mis en oeuvre à l'étape (i) est avantageusement un lit d'air fluidisé (LAF) qui autorise un « sprayage » et un séchage simultanés, assurant de ce fait une protection par enrobage. Par ailleurs ce procédé favorise une meilleure conservation de la composition selon l'invention qui est plus stable au cours du temps, au regard en particulier d'une meilleure préservation de la viabilité de la biomasse, même à température ambiante.

Le procédé décrit dans l'invention présente encore les caractéristiques complémentaires ou alternatives suivantes :
- de préférence, la teneur en matière sèche de bactéries pulvérisées à l'étape (ii) du procédé est comprise entre 10 et 26 % et de préférence entre 13 et 26 % en matière sèche de la matière sèche totale de la composition (P/P).
- Les étapes ii et iii du procédé sont de préférence simultanées.
- Le procédé comporte avantageusement en outre une étape au cours de laquelle le support enrobé est revêtu par pulvérisation d'une crème de levure et/ou d'un ou plusieurs composés choisis dans le groupe constitué par des hydrocolloïdes tels que gomme arabique, gomme de caroube, gomme de guar, gellane, xanthane, alginate, cellulose ; des amidons tels que amidon natif, amidon prégélatinisé, amidons modifiés ; des dextrines telles que maltodextrines ; des mono et disaccharides tels que glucose, tréhalose, saccharose ; seuls ou en mélange.
- Ce « sursprayage » par dépôt d'une couche protectrice permet d'améliorer la conservation dans les conditions sous air et à 20°C, en particulier le « stress oxygène » subi par la composition de l'invention est fortement réduit du fait de cette protection

La présente invention a en outre pour objets un activateur de fermentation de type « starter » tel que décrit à l'une quelconque des revendications 17, 19 à 21, et un probiotique tel que décrit à la revendication 18.

La présente invention décrit encore :
- Un « **Activateur de fermentation** » ou « **Starter** » comportant la composition de l'invention ou telle qu'obtenue selon le procédé de l'invention, en particulier de type ferment panaire ou de type ferment de vinification ou encore de type ferment laitier et
- Un « **Probiotique** » comportant la composition de l'invention ou telle qu'obtenue selon le procédé de l'invention.

En particulier l'invention procure une composition aux propriétés d'intérêt particulièrement recherchées dans l'art.

En effet, les recherches des inventeurs ont permis la mise au point d'une composition comportant un support permettant d'augmenter d'une manière surprenante la biomasse microbienne vivante qui y est pulvérisée pour atteindre une concentration microbienne notamment bactérienne encore jamais atteinte à ce jour ladite composition restant stable au cours du temps. Les inventeurs ont également observé, de manière surprenante, une amélioration substantielle des performances acidifiantes de ladite composition par rapport aux produits équivalents existants, ainsi qu'une excellente survie, même après 1 an de conservation à température ambiante ou en conservation sous air.

### EXEMPLES DE REALISATION

La présente invention va maintenant être décrite en détails dans ses autres caractéristiques et avantages au moyen d'exemples de réalisation donnés à titre purement illustratif et non limitatif et en référence aux Tableaux et Dessins annexés.

### I- MATERIELS ET METHODES

### Tests d'évaluation

**A] Test acidification au maltose**
1) Matériel et réactif :
- Bécher de 250 ml
- Bain-marie avec système agitant, température de consigne à 35°C
- pH mètre à enregistreur
- Milieu Maltose + sels :

| **Ingrédient** | **(g)** |
|---|---|
| eau distillée | 1000 |
| maltose, H₂O | 28,25 |
| K₂HPO₄ | 2 |
| MgSO₄, 7H₂O | 0,37 |
| MnSO₄, H₂O | 0,055 |

S'il n'est pas utilisé dans la journée, ce milieu doit être stérilisé.
- HCl 1N.
2) Mode opératoire milieu maltose + sels :
- Mettre le bain marie à chauffer à 35°C
- Etalonner le pH-mètre avec les tampons pH et les sondes à température
- Mettre 150 ± 0,1g de milieu dans le bécher de 250 ml
- Introduire le bécher dans le bain-marie, placer un barreau aimanté de 25 mm, et
- Lancer l'agitation à 500 r.p.m.
- Placer l'électrode du pHmètre dans le milieu
- Peser l'échantillon :
   - Peser 1 g de composition ou 10 g de témoin pour 150 ml de milieu
   - Démarrer le chronomètre et noter le pH initial
   - Après 5 mn, ajouter l'échantillon en pluie fine en évitant la formation de grumeaux.
   - Attendre ½ h et ajouter l'HCl 1 N pour descendre le pH à 6,20 ± 0,05
   - Enregistrer le pH pendant environ 20 heures et noter le pH à t=3h.

**B] Test de viabilité**
1) Matériel :
   - Milieu **microbiologique : M.R.S. agar de DIFCO**
   - **Actidion**e à 1,5 % stérilisée
   - Solution de pourpre de bromocrésol à 4 %
   - Boîtes de Pétri stériles de 90 mm
   - Etuve à 30°C
   - Jarres anaérobies + sachets pour anaérobiose Gen-box
   - Pipettes plastiques stériles
   - Etaleurs stériles
2) Technique :
   - Diluer 1 g d'échantillon qsp 100 ml d'eau stérile
   - Bien homogénéiser ; cette préparation est la dilution 10⁻¹
   - Faire des dilutions successives jusqu'à 10⁻⁹ dans des tubes d'eau stérile
   - Etaler 0,1 ml en surface sur M.R.S. en boîte de Pétri avec les dilutions adéquates
3) Lecture :
   - Incuber les boîtes de Pétri dans une jarre anaérobie, dans une étuve à 30°C pendant 24 à 72 h
   - Compter le nombre de colonies apparues sur les boîtes et déterminer le nombre d'Unités Formant Colonies (U.F.C.) par mL (ou par g) en fonction de la dilution

**C] Mesure du pH et du T.T.A. (Acidité Totale Titrable)**
- Prélever 10 ± 0,1g de mie dans un bécher de 250 ml.
- Préparer un volume de 100 ml d'eau distillée à température ambiante.
- Ajouter environ 40 ml d'eau distillée dans le bécher et mélanger jusqu'à homogénéisation (à l'aide d'un mélangeur à haute vitesse à rotor/stator type Ultraturrax si nécessaire). Compléter avec le reste de l'eau en s'en servant pour le rinçage des instruments de mélange.
- Ajouter un barreau aimanté et placer le bécher sur une plaque agitante.
- Mesurer le pH (attendre la stabilisation du pH qui doit durer au moins une minute).
- Noter le pH.
- A l'aide d'une burette de 15 ml graduée à 0,1 ml près, verser une solution de NaOH N/10 jusqu'à pH = 6,6 ± 0.1, attendre 5 mn, réajuster le pH jusqu'à stabilisation à pH 6,6 ± 0,1 pendant 1 minute.
- Noter le volume ajouté en ml (= T.T.A.).

**D] Témoin du commerce**
Témoin : Saf Levain LV1, Lesaffre International S.A.R.L. 137 rue Gabriel Péri à 59700 Marcq-en-Baroeul, France.
Ce starter contient 5x10⁹ U.F.C. de bactéries/g de type *Lactobacillus casei.*

**E] Types de supports granulaires**
- Levure Sèche Instantanée : Saf Instant, S.I.Lesaffre, 137 rue Gabriel Péri à 59700 Marcq-en-Baroeul, France.
- Semoule de blé dur claire fine, biologique (Moulin des moines Meckert-Diemer S.A. 101, route de Wingersheim à 67170 Krautwiller) surséchée en séchoir à lit d'air fluidisé, par batch de 25 kg pendant 20 min dans un flux d'air de 1000 m³/h à 78°C.

**F] Types de bactéries**
- *Lactobacillus casei* : CNCM MA43/6V

### II- EXEMPLES

### EXEMPLE 1 : Composition selon l'invention

### Exemple 1.1 : Préparation d'une crème de bactéries

- La souche de bactérie *Lactobacillus casei* est propagée en fermenteur selon un procédé classique bien connu par ailleurs, employant un milieu M.R.S. pour les pré-cultures et la culture finale, tels que décrits dans « BERGEY'S Manual of systematic bacteriology - volume 2 », SNEATH, P.H.A. ; MAIR, N.S. ; SHARPE, M.E. and HOLT, J.G. (Eds), WILLIAMS AND WILKINS(Publ), 1986, Baltimore.
- On obtient en fin de fermentation des concentrations cellulaires d'environ 10¹⁰ U.F.C./ml (U.F.C. = nombre de cellules pouvant se reproduire par unité, en l'occurrence par ml) ; le moût de fermentation est ensuite centrifugé pour fournir une crème de bactéries à environ 20 % de matière sèche totale et environ 1,5x10¹¹ U.F.C./ml.
- Cette crème est conservée au froid (4°C) en attendant d'être utilisée pour l'étape suivante. Ce temps d'attente n'excède pas quelques heures.

### Exemple 1.2 : Préparation du support enrobé par pulvérisation selon le procédé de l'invention de la crème telle qu'obtenue à l'Exemple 1.1

- La crème de bactéries de l'étape 1 est sprayée sur le support granulaire Levure Sèche Instantanée et séchée dans un courant d'air chaud.
- 425 g de levure Saf-Instant à 95,5 % de matière sèche sont introduits dans un Lit d'Air Fluidisé Glatt GPCG1.1. L'appareil est en configuration Wurster et équipé d'une buse bi-fluide de pulvérisation de 0,8 mm en position bottom.
- 690 g de crème de bactéries à 22,0 % de matière sèche sont ainsi déposés sur le support et les paramètres de fonctionnement de l'appareil (débit et température de l'air de fluidisation, débit de la suspension de sprayage) sont choisis pour que la température du produit, à tout instant de l'opération, soit en moyenne de 39,2°C.
- La durée du sprayage et du séchage dans le Lit d'Air Fluidisé est ainsi de 124 mn.

### Exemple 1.3 : Composition selon l'invention (support + bactéries)

- Dans les conditions décrites ci-dessus on obtient une composition finale dénommée « SPRAY_A » à 5,3 % d'humidité et dont la teneur en bactéries est de 27,2 % matière sèche/matière sèche totale.
- Ladite composition contient initialement après séchage 5,0x10¹⁰ U.F.C. de bactéries/g.

### RESULTATS

**Tableau 1.a : Population bactérienne du SPRAY_A en conservation à 20°C**

| | initiale | 3 mois | 6 mois | 12 mois |
|---|---|---|---|---|
| | U.F.C./g | U.F.C./g | U.F.C./g | U.F.C./g |
| Composition conservée sous air | 5,0E+10 | 1,9E+10 | 2,8E+10 | 1,1E+10 |
| Composition conservée sous vide | 5,0E+10 | 2,5E+10 | 8,0E+10 | 5,6E+10 |

On note dans le Tableau 1.a la très faible perte de population bactérienne vivante en conservation pendant 1 an à 20°C en conservation sous air et l'absence de perte de biomasse bactérienne vivante en conservation sous vide - l'augmentation apparente de la biomasse en conservation est un artefact lié à l'incertitude de la méthode analytique utilisée.

Comme illustré à la Figure 1, on observe une meilleure acidification avec la composition « SPRAY_A » de l'invention, même après une conservation pendant 1 an à une température de 20°C par comparaison avec le témoin commercial, conservé dans des conditions optimales (-20°C sous vide) et ceci à biomasse bactérienne mise en oeuvre équivalente.

Comme indiqué au Tableau 1.b ces résultats sont particulièrement avantageux par comparaison avec ceux obtenus avec le Témoin (Saf Levain LV1) et ceci en condition de stress de température (conservation à 20°C) ou de stress oxydatif (conservation sous air).

**Tableau 1.b : Résultats du test d'acidification du SPRAY_A en conservation à 20°C**

| | initial | 3 mois | 6 mois | 12 mois |
|---|---|---|---|---|
| | pH après 3 h | pH après 3 h | pH après 3 h | pH après 3 h |
| **10g de Témoin** | 5,7 | 5,7 | 5,7 | 5,7 |
| 1g de composition conservée sous air | 4,15 | 5,15 | 5,19 | 5,18 |
| 1g de composition conservée sous vide | 4,15 | 4,72 | 4,71 | 4,61 |

Ils démontrent l'intérêt du sprayage d'un fort taux de bactéries selon le procédé de l'invention par rapport aux procédés connus, car il améliore la conservation de la biomasse bactérienne vivante et de son pouvoir acidifiant en condition de stress de température ou de présence d'oxygène.
A noter que des résultats similaires ont été obtenus avec une semoule de blé dur.

### EXEMPLE 2 : Préparation d'une composition « sursprayée » selon la présente invention comportant un support enrobé à savoir un support et une couche superficielle de protection

La crème de bactéries, obtenue suivant un mode opératoire identique à celui de l'étape 1 de l'Exemple 1 est sprayée sur le support granulaire Levure Sèche Instantanée et est séchée dans un courant d'air chaud. La composition intermédiaire résultante est ensuite enrobée par une couche protectrice déposée par sursprayage d'une suspension (crème) de levures.

### Etape 1 : sprayage de la crème de bactéries (composition SPRAY_B)

La composition à sursprayer est obtenue dans les conditions suivantes : 935 g de crème de bactéries à 20,6 % de matières sèches sont déposés sur 600 g de support Saf-Instant. L'opération s'effectue dans un Lit d'Air Fluidisé dans les mêmes conditions qu'à l'Exemple 1.
On obtient une composition à sursprayer « SPRAY_B » à 5,2 % d'humidité et dont la teneur en bactéries est de 25,2 % matière sèche/matière sèche totale.

### Etape 2 : sursprayage par une crème de levures (composition SPRAY_C)

500 g de la composition précédente « SPRAY_B » sont introduits dans le Lit d'Air Fluidisé Glatt GPCG1.1, disposé en configuration Wurster et équipé d'une buse bi-fluide de pulvérisation de 0,8 mm en position bottom.
235 g d'une crème de levures (*Saccharomyces cerevisiae*) à 24 % de matières sèches sont sursprayés sur la composition « SPRAY_B » et les paramètres de fonctionnement de l'appareil (débit et température de l'air de fluidisation, débit de la suspension de sprayage) sont choisis pour que la température du produit, à tout instant de l'opération, soit en moyenne de 39,0°C. La durée de cette opération est de 37 minutes.
On obtient finalement une composition « SPRAY_C » protégée par une couche de levures, dont la teneur en humidité est de 4,3 % et qui contient 22,5 % de matière sèche bactéries/matière sèche totale.

### RESULTATS

**Tableau 2.a : Population bactérienne du SPRAY_C en conservation**

| | initiale | 3 mois | 6 mois | 12 mois |
|---|---|---|---|---|
| | U.F.C./g | U.F.C./g | U.F.C./g | U.F.C./g |
| Composition conservée sous air à 4°C | 1,5E+10 | 1,5E+10 | 2,1E+10 | 1,6E+10 |
| Composition conservée sous vide à 20°C | 1,5E+10 | 1,1E+10 | 1,4E+10 | 1,4E+10 |

On note dans le Tableau 2.a l'absence de perte significative de population bactérienne vivante en conservation pendant 1 an à 4°C en conservation sous air et l'absence de perte de biomasse bactérienne vivante en conservation à 20°C sous vide.

Comme illustré à la Figure 2, on observe une meilleure acidification avec la composition « SPRAY_C » selon l'invention, même après une conservation pendant 1 an à une température positive (4°C) par comparaison au témoin commercial, conservé dans des conditions optimales (-20°C sous vide) et ceci à biomasse bactérienne mise en oeuvre équivalente.

Comme indiqué au Tableau 2.b, ces résultats sont particulièrement avantageux par comparaison avec ceux obtenus avec le Témoin (Saf Levain LV1) et ceci soit en condition de stress de température (conservation à 20°C) ou de stress oxydatif (conservation sous air). Ces résultats démontrent l'intérêt d'un sursprayage comme protection de la biomasse d'intérêt.

**Tableau 2.b : Résultats du test d'acidification du SPRAY_C en conservation à 4°C**

| | initial | 3 mois | 6 mois | 12 mois |
|---|---|---|---|---|
| | pH après 3 h | pH après 3 h | pH après 3 h | pH après 3 h |
| **10g de Témoin** | 5,7 | 5,7 | 5,7 | 5,7 |
| 1g de composition conservée sous air | 4,78 | 5,19 | 5,56 | 5,33 |
| 1g de composition conservée sous vide | 4,78 | 5,09 | 5,58 | 5,31 |

### EXEMPLE 3 : Applications

### FERMENT PANAIRE

Fabrication d'un pain au levain à partir de la composition conforme à l'invention et décrite à l'Exemple 1.

On réalise deux essais de panification suivant la formule et le procédé décrit ci-après.
Essai 1 : pain au levain fabriqué avec le témoin Saf Levain LV1 à 3 mois de conservation sous vide à -20°C.
Essai 2 : pain au levain fabriqué avec la composition de l'Exemple 1 à 1 an de conservation sous vide à 20°C (température ambiante).
1)

**Fabrication des levains :**

| **Formule** | Essai 1 : levain 1 | Essai 2 : levain 2 |
|---|---|---|
| Ingrédients | % de la farine mise en oeuvre | % de la farine mise en oeuvre |
| Farine de blé T55 | 100 | 100 |
| Eau | 54 | 54 |
| Sel | 1,5 | 1,5 |
| Saf Levain LV1 | 0,5 | |
| Composition suivant l'Exemple 1 | | 0,05 |

Ces deux pâtes, ci-après dénommées « levain » sont laissées à fermenter pendant 20 h à 30°C.
2) Fabrication des pains :

**Composition de la pâte finale**

| | Essai 1 : pâte 1 | Essai 2 : pâte 2 |
|---|---|---|
| | % de la farine mise en oeuvre | % de la farine mise en oeuvre |
| Farine de blé T55 | 100 | 100 |
| eau | 64 | 64 |
| Sel | 1,8 | 1,8 |
| Levain 1 | 30 | |
| Levain 2 | | 30 |
| Levure pressée Hirondelle bleue Lesaffre | 0,2 | 0,2 |

### Schéma de fabrication

**pétrissage Artofex**

| | |
|---|---|
| vitesse lente (40 r.p.m.) | 2 mn |
| vitesse rapide (60 r.p.m.) | 13 mn |
| température pâte | 26°C |

**pointage**

| | |
|---|---|
| temps | 160 mn |
| température | ambiante |
| humidité | ambiante |

**pesage**

| | |
|---|---|
| poids | 500 g |
| temps | 10 mn |

**détente**

| | |
|---|---|
| temps | 20 mn |
| température | ambiante |
| humidité | ambiante |

**apprêt**

| | |
|---|---|
| temps | 3 h |
| température | ambiante |
| humidité | ambiante |

**cuisson four à sole**

| | |
|---|---|
| temps | 45 mn |
| température | 225°C |
| humidité | Buée : 1+1 |

3)

**Résultats :**

| | Volume spécifique cm³/g | pH de la mie | T.T.A. de la mie ml (10 g) | Appréciation organoleptique |
|---|---|---|---|---|
| | cm³/g | | ml (10g) | |
| Essai 1 | 4.1 | 4.4 | 3.4 | +++ |
| Essai 2 | 3.9 | 4.3 | 4 | +++ |

Ces résultats, jugés similaires tant au niveau analytique qu'au niveau de la dégustation, montrent l'intérêt de la composition de l'Exemple 1 par rapport à un témoin du commerce. En effet, bien que conservée pendant un an à température ambiante et ajoutée à une dose 10 fois moindre par rapport au témoin (essai 1), la composition de l'Exemple 1, conforme à l'invention, produit un pain au caractéristiques similaires à celles d'un starter du commerce conservé seulement 3 mois dans des conditions optimales (-20°C sous vide).

## Revendications

1. Composition comprenant une levure sèche active enrobée de manière régulière par une biomasse bactérienne constituée de bactéries appartenant au genre *Lactobacillus, Pediococcus, Streptococcus, Leuconostoc, Lactococcus, Bifidobacterium, Propionibacterium* et/ou *Bacillus,* ladite biomasse représentant de 10 à 30 % en matière sèche de la matière sèche totale de la levure enrobée (P/P).

2. Composition selon la revendication 1, **caractérisée en ce que** ladite biomasse représente de 13 à 26 % en matière sèche de la matière sèche totale de la levure enrobée.

3. Composition selon l'une quelconque des revendications 1 à 2, **caractérisée en ce que** la levure enrobée comprend en outre une couche superficielle de protection comprenant au moins un composé choisi parmi les hydrocolloïdes, les gommes, la dextrine, les poly, di ou monosaccharides et leurs dérivés.

4. Composition selon l'une quelconque des revendications précédentes, **caractérisée en ce que** ladite levure se présente sous la forme de granules et/ou de sphérules.

5. Composition selon la revendication 4, **caractérisée en ce que** lesdites sphérules présentent un diamètre moyen d(0,5) compris entre 150 µm et 2000 µm, de préférence entre 150 µm et 1200 µm et encore plus préférentiellement entre 150 µm et 700 µm.

6. Composition selon la revendication 4, **caractérisée en ce que** lesdits granules présentent une longueur moyenne comprise entre 1,8 et 2,2 mm et un diamètre moyen compris entre 0,4 et 0,7 mm.

7. Composition selon l'une quelconque des revendications précédentes, **caractérisée en ce que** la levure comporte un additif ou auxiliaire de fabrication choisi dans le groupe constitué par les mono et diglycérides d'acides gras modifiés, des esters d'acides gras et de sorbitan, tel que le monostéarate de sorbitan, des esters d'acides gras et de glycérol, des esters d'acides gras et de propylène glycol, de la méthylcellulose, de la carboxyméthylcellulose, de l'hydroxypropylcellulose et/ou un mélange de ces derniers.

8. Composition selon l'une quelconque des revendications précédentes, **caractérisée en ce que** la levure présente un taux de matières sèches actives supérieur à 94 %, de préférence supérieur à 96 %.

9. Composition selon l'une quelconque des revendications précédentes, **caractérisée en ce que** la levure est du genre Saccharomyces comprenant notamment l'espèce *Saccharomyces chevalieri*.

10. Composition selon la revendication 1, **caractérisée en ce que** les bactéries sont choisies dans le groupe comprenant les espèces : *Lactobacillus plantarum, Lactobacillus brevis, Lactobacillus casei, Lactobacillus paracasei, Lactobacillus sanfrancisco, Lactobacillus amylovorum, Lactobacillus kefir, Lactobacillus pentosaceus, Lactobacillus acidilactici, Lactobacillus rhamnosus, Leuconostoc oenos, Leuconostoc mesenteroïdes et Bacillus subtilis.*

11. Composition selon l'une quelconque des revendications précédentes, **caractérisée en ce que** la levure enrobée comprend en outre une couche protectrice qui consiste en une crème de levures, de préférence une crème de *S*. *chevalieri*.

12. Composition selon l'une quelconque des revendications 1 à 11, **caractérisée en ce qu'**elle présente un taux de mortalité bactérien inférieur ou égal à 0,5 Log U.F.C./g après une conservation d'un an à une température de 20 °C sous vide et/ou après une conservation d'un an à une température de 4 °C sous air.

13. Composition selon l'une des revendications 1 à 12, **caractérisée en ce qu'**elle présente après une conservation sous vide de 1 an à une température de 20°C, lors du test d'acidification sur milieu au maltose, une diminution de pH de 6,5 à 5,7 après 3h.

14. Procédé de préparation d'une composition selon l'une des revendications 1 à 13 comprenant les étapes suivantes consistant à :
i- introduire une levure apte à être enrobée dans un mélangeur traversé par un courant ascendant d'air chaud,
ii- pulvériser une suspension de biomasse bactérienne comprenant plus de 5 % en matière sèche de bactéries, ladite biomasse bactérienne étant constituée de bactéries appartenant au genre *Lactobacillus, Pediococcus, Streptococcus, Leuconostoc, Lactococcus, Bifidobacterium, Propionibacterium* et/ou *Bacillus,*
iii- sécher par courant d'air chaud dont la température et le débit sont fixés de manière à ce que la température de ladite biomasse ne dépasse pas 40°C, les étapes (ii) et (iii) étant simultanées,
iv- récupérer la levure apte à être enrobée, et
v- procurer ladite composition.

15. Procédé selon la revendication 14, **caractérisé en ce que** la teneur en bactéries est comprise entre 10 et 26 % en matière sèche et de préférence entre 13 et 26 % en matière sèche de la matière sèche totale de la composition (P/P).

16. Procédé selon la revendication 14 ou 15, **caractérisé en ce qu'**il comporte en outre une étape au cours de laquelle la levure est enrobée par pulvérisation d'une crème de levures et/ou d'un composé choisi dans le groupe constitué par des hydrocolloïdes tels que gomme arabique, gomme de caroube, gomme de guar, gellane, xanthane, alginate, cellulose ; des amidons tels que amidon natif, amidon prégélatinisé, amidons modifiés ; des dextrines telles que maltodextrines ; des mono et disaccharides tels que glucose, tréhalose, saccharose ; seuls ou en mélange.

17. Activateur de fermentation de type Starter comportant une composition selon l'une quelconque des revendications 1 à 13.

18. Probiotique comportant une composition selon l'une quelconque des revendications 1 à 13.

19. Activateur selon la revendication 17, **caractérisé en ce qu'**il représente un ferment panaire.

20. Activateur selon la revendication 17, **caractérisé en ce qu'**il représente un ferment de vinification.

21. Activateur selon la revendication 17, **caractérisé en ce qu'**il représente un ferment laitier.

## Patentansprüche

1. Zusammensetzung, umfassend eine aktive Trockenhefe, die gleichmäßig mit einer bakteriellen Biomasse umhüllt ist, die aus Bakterien besteht, die zur Gattung *Lactobacillus, Pediococcus, Streptococcus*, *Leuconostoc, Lactococcus, Bifidobacterium, Propionibacterium* und/oder *Bacillus* gehören,
wobei die Biomasse 10 bis 30 % Trockensubstanz der gesamten Trockensubstanz der umhüllten Hefe ausmacht (Gew./Gew.) .

2. Zusammensetzung gemäß Anspruch 1, **dadurch gekennzeichnet, dass** die Biomasse 13 bis 26 % Trockensubstanz der gesamten Trockensubstanz der umhüllten Hefe ausmacht.

3. Zusammensetzung gemäß einem der Ansprüche 1 bis 2, **dadurch gekennzeichnet, dass** die umhüllte Hefe ferner eine Oberflächen-Schutzschicht umfasst, die mindestens eine Verbindung umfasst, die aus Hydrokolloiden, Gummen, Dextrinen, Poly-, Di- oder Monosacchariden und Derivaten davon ausgewählt ist.

4. Zusammensetzung gemäß einem der vorherigen Ansprüche, **dadurch gekennzeichnet, dass** die Hefe in Form von Granulaten und/oder in Form von Kügelchen vorliegt.

5. Zusammensetzung gemäß Anspruch 4, **dadurch gekennzeichnet, dass** die Kügelchen einen mittleren Durchmesser d(0,5) zwischen 150 µm und 2000 µm, vorzugsweise zwischen 150 µm und 1200 µm und noch mehr bevorzugt zwischen 150 µm und 700 µm aufweisen.

6. Zusammensetzung gemäß Anspruch 4, **dadurch gekennzeichnet, dass** die Granulate eine mittlere Länge zwischen 1,8 und 2,2 mm und einen mittleren Durchmesser zwischen 0,4 und 0,7 mm aufweisen.

7. Zusammensetzung gemäß einem der vorherigen Ansprüche, **dadurch gekennzeichnet, dass** die Hefe ein Additiv oder ein Verarbeitungshilfsmittel umfasst, das ausgewählt ist aus der Gruppe bestehend aus Mono- und Diglyceriden modifizierter Fettsäuren, Sorbitan-Fettsäureestern, wie z.B. Sorbitanmonostearat, Glycerol-Fettsäureestern, Estern aus Fettsäuren und Propylenglycol, Methylcellulose, Carboxymethylcellulose, Hydroxypropylcellulose und/oder einer Mischung letzterer.

8. Zusammensetzung gemäß einem der vorherigen Ansprüche, **dadurch gekennzeichnet, dass** der Anteil an aktiver Trockensubstanz der Hefe mehr als 94 %, vorzugsweise mehr als 96 % beträgt.

9. Zusammensetzung gemäß einem der vorherigen Ansprüche, **dadurch gekennzeichnet, dass** die Hefe der Gattung der Zuckerhefen (*Saccharomyces*), die insbesondere die Spezies *Saccharomyces chevalieri* umfasst, angehört.

10. Zusammensetzung gemäß Anspruch 1, **dadurch gekennzeichnet, dass** die Bakterien ausgewählt sind aus der Gruppe umfassend die Spezies: *Lactobacillus plantarum, Lactobacillus brevis, Lactobacillus casei, Lactobacillus paracasei, Lactobacillus sanfrancisco, Lactobacillus amylovorum, Lactobacillus kefir, Lactobacillus pentosaceus, Lactobacillus acidilactici, Lactobacillus rhamnosus, Leuconostoc oenos, Leuconostoc mesenteroides et Bacillus subtilis.*

11. Zusammensetzung gemäß einem der vorherigen Ansprüche, **dadurch gekennzeichnet, dass** die umhüllte Hefe ferner eine Schutzschicht umfasst, die aus einer Hefecreme, vorzugsweise einer Creme von *S. chevalieri* besteht.

12. Zusammensetzung gemäß einem der Ansprüche 1 bis 11, **dadurch gekennzeichnet, dass** sie eine Bakteriensterblichkeitsrate von weniger als 0,5 log KBE/g nach einer Lagerung von einem Jahr bei einer Temperatur von 20 °C unter Vakuum und/oder nach einer Lagerung von einem Jahr bei einer Temperatur von 4 °C unter Luft aufweist.

13. Zusammensetzung gemäß einem der Ansprüche 1 bis 12, **dadurch gekennzeichnet, dass** sie nach einer Lagerung unter Vakuum von einem Jahr bei einer Temperatur von 20 °C, gemäß dem Ansäuerungstest auf einem Maltosemilieu, eine Verringerung des pH von 6,5 auf 5,7 nach 3 Stunden aufweist.

14. Verfahren zur Herstellung einer Zusammensetzung gemäß einem der Ansprüche 1 bis 13, umfassend die folgenden Schritte, bestehend aus:
i- Einführen einer zum Umhüllen geeigneten Hefe in einen Mischer, der von einem aufsteigenden Heißluftstrom durchströmt wird.
ii- Zerstäuben einer Suspension einer bakteriellen Biomasse, die mehr als 5 % Trockensubstanz an Bakterien umfasst, wobei die bakterielle Biomasse aus Bakterien besteht, die zur Gattung *Lactobacillus, Pediococcus, Streptococcus, Leuconstoc, Lactococcus, Bifidobacterium, Propionibacterium* und/oder *Bacillus* gehören,
iii- Trocknen durch einen Heißluftstrom, wobei die Temperatur und der Durchsatz so festgelegt werden, dass die Temperatur der Biomasse 40 °C nicht übersteigt, wobei die Schritte (ii) und (iii) gleichzeitig erfolgen,
iv- Rückgewinnen der zum Umhüllen geeigneten Hefe, und v- Bereitstellen der Zusammensetzung.

15. Verfahren gemäß Anspruch 14, **dadurch gekennzeichnet, dass** der Gehalt an Bakterien zwischen 10 und 26 % Trockensubstanz und vorzugsweise zwischen 13 und 26 % Trockensubstanz der gesamten Trockensubstanz der Zusammensetzung (Gew./Gew.) beträgt.

16. Verfahren gemäß Anspruch 14 oder 15, **dadurch gekennzeichnet, dass** es ferner einen Schritt umfasst, bei dem die Hefe durch Zerstäuben einer Hefecreme und/oder einer Verbindung umhüllt wird, die ausgewählt wird aus der Gruppe bestehend aus Hydrokolloiden wie z.B. Gummiarabikum, Johannisbrotgummi, Guargummi, Gellan, Xanthan, Alginat, Cellulose; Stärken wie z.B. natürliche Stärken, vorgelatinierte Stärken, modifizierte Stärken; Dextrinen wie z.B. Maltodextrine; Mono- und Disacchariden wie z.B. Glucose, Trehalose, Saccharose; einzeln oder in einer Mischung.

17. Aktivierungsmittel für die Fermentierung des Starter-Typs, das eine Zusammensetzung gemäß einem der Ansprüche 1 bis 13 umfasst.

18. Probiotikum, das eine Zusammensetzung gemäß einem der Ansprüche 1 bis 13 umfasst.

19. Aktivierungsmittel gemäß Anspruch 17, **dadurch gekennzeichnet, dass** es ein Brotgärungsmittel ist.

20. Aktivierungsmittel gemäß Anspruch 17, **dadurch gekennzeichnet, dass** es ein Gärungsmittel zur Weinherstellung ist.

21. Aktivierungsmittel gemäß Anspruch 17, **dadurch gekennzeichnet, dass** es ein Milchgärungsmittel ist.

## Claims

1. A composition comprising an active dry yeast evenly coated with a bacterial biomass consisting of bacteria belonging to the genera *Lactobacillus, Pediococcus, Streptococcus, Leuconostoc, Lactococcus, Bifidobacterium, Propionibacterium* and/or *Bacillus,* said biomass representing from 10% to 30% by dry matter of the total dry matter of the coated yeast (W/W).

2. The composition as claimed in claim 1, **characterized in that** said biomass represents from 13% to 26% by dry matter of the total dry matter of the coated yeast.

3. The composition as claimed in either one of claims 1 and 2, **characterized in that** the coated yeast also comprises a superficial protective layer comprising at least one compound chosen from hydrocolloids, gums, dextrins, poly-, di- or monosaccharides, and derivatives thereof.

4. The composition as claimed in any one of the preceding claims, **characterized in that** said yeast is in the form of granules and/or of spherules.

5. The composition as claimed in claim 4, **characterized in that** said spherules have a mean diameter d(0.5) of between 150 µm and 2000 µm, preferably between 150 µm and 1200 µm and even more preferentially between 150 µm and 700 µm.

6. The composition as claimed in claim 4, **characterized in that** said granules have a mean length of between 1.8 and 2.2 mm and a mean diameter of between 0.4 and 0.7 mm.

7. The composition as claimed in any one of the preceding claims, **characterized in that** said yeast comprises a production additive or aid chosen from the group consisting of modified fatty acid monoglycerides and diglycerides, fatty acid esters of sorbitan, such as sorbitan monostearate, fatty acid esters of glycerol, fatty acid esters of propylene glycol, methylcellulose, carboxymethylcellulose, hydroxypropylcellulose and/or a mixture of the latter.

8. The composition as claimed in any one of the preceding claims, **characterized in that** said yeast has an active dry matter content of greater than 94%, preferably greater than 96%.

9. The composition as claimed in any one of the preceding claims, **characterized in that** said yeast is of the *Saccharomyces* genus comprising in particular the *Saccharomyces chevalieri* species.

10. The composition as claimed in claim 1, **characterized in that** said bacteria are chosen from the group comprising the species: *Lactobacillus plantarum, Lactobacillus brevis, Lactobacillus casei, Lactobacillus paracasei, Lactobacillus sanfrancisco, Lactobacillus amylovorum, Lactobacillus kefir, Lactobacillus pentosaceus, Lactobacillus acidilactici, Lactobacillus rhamnosus, Leuconostoc oenos, Leuconostoc mesenteroïdes* and *Bacillus subtilis.*

11. The composition as claimed in any one of the preceding claims, **characterized in that** the coated yeast also comprises a protective layer which consists of a cream yeast, preferably a cream of S. *chevalieri.*

12. The composition as claimed in any one of claims 1 to 11, **characterized in that** it has a bacterial mortality rate of less than or equal to 0.5 Log CFU/g after storage for one year at a temperature of 20°C under vacuum and/or after storage for one year at a temperature of 4°C under air.

13. The composition as claimed in any one of claims 1 to 12, **characterized in that** it exhibits, after storage under vacuum for 1 year at a temperature of 20°C, in the acidification test on medium comprising maltose, a decrease in pH from 6.5 to 5.7 after 3 h.

14. A process for preparing a composition as claimed in one of claims 1 to 13, comprising the following steps consisting in:
i- introducing a yeast capable of being coated into a mixer through which an ascending stream of hot air passes,
ii- spraying a suspension of bacterial biomass comprising more than 5% by dry matter of bacteria, said bacterial biomass consisting of bacteria belonging to the genera *Lactobacillus, Pediococcus, Streptococcus, Leuconostoc, Lactococcus, Bifidobacterium, Propionibacterium* and/or *Bacillus,*
iii- drying by means of a stream of hot air, the temperature and the flow rate of which are fixed such that the temperature of said biomass does not exceed 40°C, steps ii and iii being simultaneous,
iv- recovering the yeast capable of being coated, and
v- obtaining said composition.

15. The process as claimed in claim 14, **characterized in that** the content of bacteria is between 10% and 26% by dry matter and preferably between 13% and 26% by dry matter of the total dry matter of the composition (W/W).

16. The process as claimed in claim 14 or 15, **characterized in that** it also comprises a step during which the yeast is coated by spraying with a cream yeast and/or with a compound chosen from the group consisting of hydrocolloids such as gum arabic, locust bean gum, guar gum, gellan, xanthan, alginate or cellulose; starches such as native starch, pregelatinized starch or modified starches; dextrins such as maltodextrins; monosaccharides and disaccharides such as glucose, trehalose or sucrose; alone or as a mixture.

17. A fermentation activator of starter type containing a composition as claimed in any one of claims 1 to 13.

18. A probiotic containing a composition as claimed in any one of claims 1 to 13.

19. The activator as claimed in claim 17, **characterized in that** it represents a bread ferment.

20. The activator as claimed in claim 17, **characterized in that** it represents a wine-making ferment.

21. The activator as claimed in claim 17, **characterized in that** it represents a milk ferment.
